(19) Europäisches Patentamt
European Patent Office
Office européen des brevets

(11) Publication number: **0 173 751**
**B1**

# (12) EUROPEAN PATENT SPECIFICATION

(45) Date of publication of patent specification: **31.01.90**

(51) Int. Cl.[5]: **B 01 J 13/02**

(21) Application number: **85901069.6**

(22) Date of filing: **21.02.85**

(86) International application number:
**PCT/JP85/00075**

(87) International publication number:
**WO 85/03648 29.08.85 Gazette 85/19**

## (54) Process for preparing capsules.

(30) Priority: **23.02.84 JP 33062/84**

(43) Date of publication of application:
**12.03.86 Bulletin 86/11**

(45) Publication of the grant of the patent:
**31.01.90 Bulletin 90/05**

(84) Designated Contracting States:
**FR**

(56) References cited:
**EP-A-0 152 898**
**WO-A-84/00294**
**GB-A-2 145 992**
**JP-A-57 197 214**
**US-A-3 522 346**
**US-A-3 826 755**

**CHEMICAL ABSTRACTS, vol. 94, 27th April 1981, page 389, abstract 135513f, Columbus, Ohio, US; K.D. VORLOP et al.: "Formation of spherical chitosan biocatalysts by ionotropic gelation", & BIOTECHNOL. LETT. 1981, 3(1), 9-14**

(73) Proprietor: **SNOW BRAND MILK PRODUCTS & CO., LTD.**
**1-1, Naebo-cho 6-chome Higashi-ku**
**Sapporo-shi Hokkaido 065 (JP)**

(72) Inventor: **SHIOYA, Toshiaki**
**1-505, Esteeto Kabe 4, Morooka 4-chome**
**Oume-shi Tokyo 198 (JP)**
Inventor: **SHIINOKI, Yasuhiko 1-203, Toei Kitashinagawa**
**Apaato 7, Kitashinagawa 1-chome Shinagawa-ku**
**Tokyo 140 (JP)**
Inventor: **KIMURA, Toshiaki**
**161-6, Ohaza Kamiokutomi**
**Sayama-shi Saitama 350-13 (JP)**
Inventor: **KAKO, Masatoshi**
**G302, Sayamadai Haitsu 1354-19, Irumagawa**
**Sayama-shi Saitama 350-13 (JP)**
Inventor: **HAYASHI, Hiromichi**
**K-215, Kotesashihaitsu, 3-15, Kotesashicho**
**Tokorozawa-shi, Saitama 359 (JP)**

(74) Representative: **Lecca, Jean et al**
**CABINET PLASSERAUD 84, rue d'Amsterdam**
**F-75009 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:

**CHEMICAL ABSTRACTS, vol. 97, no. 12, 1982, page 112, abstract 94239n, Columbus, Ohio, US; H. FUKUDA: "Polyelectrolyte complex consisting of ternary system, sodium dextran sulfate, sodium carboxymethyldextran and chitosan", & HIROSHIMA JOSHI DAIGAKU KASEIGAKUBU KIYO 1982, (17), 1-7**

**Description**

Technical Field

This invention relates to a process for preparing capsules making use of a soluble chitin derivative such as chitosan as a wall-forming material and their preparation process.

Background Art

As preparation processes of capsules, there have conventionally been known to bring a mixture, which has been obtained by adding a multivalent metal salt such as calcium salt to a fluid material (core liquid) adapted to make up the core parts of capsules, into contact with a solution of an alginate or low methoxyl pectin or a mixture thereof so as to form gel-like walls (Japanese Patent Publication No. 16183/1973), to drop with stirring an aqueous solution, which contains carboxymethylchitin or an N-deactylated product of a carboxymethylchitin salt, into an aqueous solution of an organic acid anhydride such as a mixed solution of acetic anhydride and acetic acid so as to disperse the former aqueous solution in the latter aqueous solution (Japanese Patent Laid-open No. 90503/1980), etc. In each of these conventionally known processes, the liquid (core liquid) making up the core parts of capsules have high ionic strength and in addition, the liquid undergoes abrupt changes in its hydrogen ion concentration in the course of formation of capsules. Accordingly, it is difficult to conduct the encapsulation under biologically mild conditions. Hence, such conventional processes are considered to be unsuitable as preparation processes of capsules which serve to enclose a biologically subtle substance therein.

Disclosure of the Invention

In the course of a research on the formation of gel-like membranes or walls suitable for the preparation of capsules, the present inventors have found that excellent gel-like membranes or walls can be formed under biologically mild conditions when a solution of chitosan, a chitin derivative, is brought into contact with a solution of a polyanionic polysaccharide and its salt, leading to completion of this invention.

EP—A—0152898 including France as a designated State and claiming a priority of February 15, 1984, describes a process for encapsulating biologically active material, wherein the material is suspended in a first aqueous solution containing a first ionic polymer and the first aqueous solution is added dropwise to a second solution containing a second ionic polymer having an ionic charge opposite from said first ionic polymer, thereby forming the walls of the encapsules which contain the first solution and the biologically active material as core material.

The first ionic polymer may be both cationic and anionic. As anionic polymers alginate, carrageenan and carboxymethyl cellulose are suggested. Among the suitable cationic polymers chitosan is preferred.

Particularly example I describes the preparation of microcapsules containing a solution of anionic polysaccharides (alginate) as core material, wherein the solution of the anionic polysaccharide is added dropwise to a solution of a soluble chitin derivative (chitosan) thereby to form gel-like walls.

According to the invention the capsules are prepared by a process including bringing the fluid material into contact under biologically mild conditions with a solution prepared by adding a soluble chitin derivative to a biologically mild aqueous solution of acetic acid and of a salt of acetic acid.

Without the salt of acetic acid, chitosan would dissolve in an acetic acid aqueous solution having a pH of about 3—4, which does not provide a biologically mild condition.

Therefore an acetate buffer solution is used to dissolve chitosan while maintaining a mild condition according to the invention.

The aqueous solution of actic acid and of a salt of said acid allows to obtain easily a mild and stable condition and to change the permeability of the gel-like walls; further it allows to obtain a considerably high strength of the walls.

More precisely, the invention allows to change the permeability of the gel-like walls of the capsules by controlling certain conditions for the formation of the gel-like walls.

The invention has therefore for an object a process for preparing capsules, which comprises preparing a fluid material, which is formed of an aqueous solution containing one of polyanionic polysaccharides and salts thereof or a mixture of two or more of the polyanionic polysaccharides and salts and adapted to form the core parts of the capsules, characterized by bringing said fluid material into contact under biologically mild conditions with a solution of a soluble chitin derivative, prepared by adding said single chitin derivative to a biologically mild aqeuous solution of acetic acid and a salt of acetic acid, whereby to form gel-like walls.

Preferably the salt is sodium acetate.

Preferably also the soluble chitin derivative is chitosan.

Best Mode for Carrying Out the Invention

The polyanionic polysaccharides or their salts useful for the formation of the gel-like walls of capsules in the present invention are polysaccharides or their salts which are converted into polyanionic polymers in aqueous solutions. Low methoxyl pectin, carageenan, carboxymethylcellulose, sodium alginate, chondroitin sulfate and the like may be mentioned by way of example. These polysaccharides may be used either singly or as a mixture.

On the other hand, the soluble chitin derivative which is also employed for the formation of gel-like walls is obtained by subjecting chitin, which is an inert substance inherently, to a chemical treatment and hence enhancing its reactivity. As a representative example, may be mentioned chitosan which is obtained by subjecting chitin to an deacetylating treatment.

Incidentally, chitin is a straight-chain homopolysaccharide formed of β (1 → 4)-bonded N-acetyl-D-glucosamine which is contained in cell walls of arthropods such as crabs, Euphausiacea and insects, fungi, etc. It occurs naturally and abundantly. However, it is considered to be an unutilized natural resource because it cannot be used as it is due to its inert nature.

However, chitin derivatives such as chitosan which is obtained by subjecting chitin to a deacetylating treatment have been rendered soluble in mild acids and have hence been imparted with reactivity. Namely, chitosan has a structural unit represented by the following general formula (I):

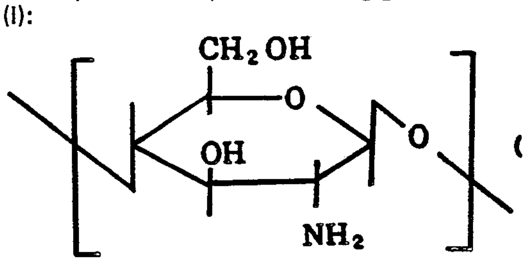

(I)

is charged in positive owing to the amino group contained in its formula, contains chitosan molecules as a polycationic polymer, and shows reactivity.

Accordingly, when an aqeous solution of the above-described polyanionic polysaccharide or its salt or a mixture thereof is brought into contact with a solution of chitosan as the above-described soluble chitin derivative, an ionic crosslinking reaction, in other words, ionic crosslinking bonding takes place between the polyanionic polysaccharide and chitosan to form a gel-like substance.

In order to achieve the contact between both of the above-described solutions in the present invention, it may be preferred to add dropwise and with stirring an aqueous solution containing the above-mentioned polyanionic polysaccharide and its salts into a chitosan solution by means of a depositor or the like. Upon the dropwise addition, the above-described crosslinking reaction takes place. Once gel-like walls have been formed by the crosslinking reaction, the solution enclosed in the walls and making up the core parts does not undergo any gellation, thereby obtaining desired capsules. Such a phenomenon may be attributed to such an assumption that once gel-like walls have been formed, the polyanionic polymer (i.e., core liquid) enclosed within the walls and chitosan molecules contained in the solution can no longer penetrate thorugh the walls and the reaction is hence not allowed to proceed any longer in the core liquid.

Upon preparation of a solution adapted to be used as a fluid material for making up the core parts of capsules, it is suitable to prepare an aqueous solution containing 0.3—1.0 wt.% of the polyanionic polysaccharides or salt or the mixture thereof in the present invention. It is particularly preferred to use carboxymethylcellulose as the polyanionic polysaccharide from the standpoint of formation of gel-like walls.

On the other hand, the chitosan solution to which the above aqueous solution is brought into contact is suitably a solution obtained by dissolving chitosan to a concentration of 0.5—1.0 wt.% in a mild acid such as acetic acid.

As mentioned above, the present invention permits the formation of gel-like capsule walls under extremely mild conditions, namely, biologically mild conditions and in a short period of time without using an organic solvent or the like. It is thus possible to add and disperse subtle biological substances or functional subtances and various other additives, which may be chosen depending what end use will be made of the resulting capsules, to the above-described fluid material adapted to form the core parts of the capsules. Hence, the present invention can provide capsules which enclose a core liquid containing various useful substances therein.

Furthermore, the present invention can achieve encapsulation, which depends on the formation of gel-like walls, in one step. This invention is therefore considered to be more advantageous from the viewpoint of preparation, compared with the known encapsulation process making use of the salt-forming crosslinking between polyanions and polycations disclosed for example in Japanese Patent Laid-open No. 197031/1982.

In addition, the present invention also allows to change the permeability of the gel-like walls of capsules by controlling certain conditions for the formation of the gel-like walls thereby imparting a fractional function to the gel-like walls.

As has been described above, the present invention has a merit that it can provide capsules, which can be used for a wide variety of applications, using a readily-available material, with simple preparation means, and in a short period of time.

Examples will be given below to describe the present invention more specifically.

## Example 1

A 0.5 wt.% aqueous solution of chitosan was prepared by adding chitosan to an aqueous solution which contained 0.28 mole/liter of acetic acid and 0.037 mole/liter of sodium acetate.

While stirring the aqueous chitosan solution at a temperature of 25°C, a 1 wt.% aqueous solution of k-carageenan which solution had been prepared on the side was dropped under pressure to the former solution through a syringe needle having an inner diameter of 0.4 mm. In 2—3 minutes after the contact of both solutions subsequent to the dropwise addition, spherical capsules of 2—3 mm in diameter were formed.

The walls of the thus-obtained capsules were transparent, and their wall thicknesses were measured to be 10—20 µm by an optical microscope. Even when those capsules were left over for a long period of time in water, the core liquid enclosed with the capsules exhibited stable fluidity and no changes were observed thereon.

Example 2

Following the procedure of Example 1, spherical capsules of about 4 mm in diameter were formed by dropping at a temperature of 35°C a 0.8 wt.% aqueous solution of carboxymethylcellulose, which had been prepared on the side, to an aqueous chitosan solution prepared in the same manner as in Example 1.

The walls of the thus-obtained capsules were transparent, and their wall thicknesses were measured to be 2—3 µm by a scanning electron microscope. In addition, it was also recognized that the strength of the walls *per se* was considerably high. The core parts enclosed within the capsules maintained stable fluidity even when the capsules were left over for a long period of time in water.

**Claims**

1. A process for preparing capsules, which comprises preparing a fluid material, which is formed of an aqueous solution containing one of polyanionic polysaccharides and salts thereof or a mixture of two or more of the polyanionic polysaccharides and salts and adapted to form the core parts of the capsules, characterized by bringing said fluid material into contact under biologicaly mild conditions with a solution of a soluble chitin derivative, prepared by adding said soluble chitin derivative to a biologicaly mild aqueous solution of acetic acid and a salt of acetic acid, whereby to form gel-like walls.

2. A process according to claim 1, wherein said salt is sodium acetate.

3. A process according to claim 1 or 2, wherein said soluble chitin derivative is chitosan.

**Patentansprüche**

1. Verfahren zur Herstellung von Kapseln, wobei ein flüssiges Material aus einer ein polyanionisches Polysaccharid und Salze davon oder ein Gemisch aus zwei oder mehreren polyanionischen Polysacchariden und Salzen davon enthaltenden wäßrigen Lösung herstellt wird, das als Kernstück der Kapseln dient, dadurch gekennzeichnet, daß man das flüssige Material unter biologisch milden Bedingungen mit einer Lösung eines löslichen Chitinderivats in Berührung bringt, wobei die Lösung durch Zugabe des löslichen Chitinderivats zu einer biologisch milden wäßrigen Lösung aus Essigsäure und einem Essigsäuresalz hergestellt wird, wodurch gelartige Wände entsehen.

2. Verfahren nach Anspruch 1, wobei das Salz Natriumacetat ist.

3. Verfahren nach Anspruch 1 oder 2, wobei das lösliche Chitinderivat Chitosan ist.

**Revendications**

1. Procédé pour préparer des capsules, qui consiste à préparer une matière fluide formée par une solution aqueuse contenant un des polysaccharides polyanioniques et de leurs sels ou un mélange de deux ou davantage de polysaccharides polyanioniques et de sels et adaptée pour former les portions de noyaux des capsules, caractérisé en ce que l'on met en contact cette matière fluide sous des conditions biologiques douces avec une solution d'un dérivé soluble de la chitine, préparée en ajoutant ledit dérivé soluble de la chitine à une solution aqueuse biologiquement douce d'acide acétique et d'un sel de l'acide acétrique, pour former des parois du type gel.

2. Procédé selon la revendication 1, dans lequel ledit sel est de l'acétate de sodium.

3. Procédé selon la revendication 1 ou 2, dans lequel le dérivé soluble de la chitine est le chitosan.